# FASCICULE DE BREVET EUROPEEN

(11) **EP 4 201 485 B1**
(45) Date de publication et mention de la délivrance du brevet: **15.05.2024**
(21) Numéro de dépôt: 22215320.7
(22) Date de dépôt: 21.12.2022
(51) Int. Cl.: A61K 36/04, A61P 33/02, A61P 33/06

(54) **COMPOSITION COMPRENANT DES EXOPOLYSACCHARIDES EXTRAITS DE PORPHYRIDIUM SP POUR LE TRAITEMENT ET/OU LA PRÉVENTION D'INFECTIONS PAR LES PARASITES DU PHYLUM APICOMPLEXA**
ZUSAMMENSETZUNG MIT EXOPOLYSACCHARIDEN AUS PORPHYRIDIUM SP ZUR BEHANDLUNG UND/ODER PRÄVENTION VON PARASITÄREN INFEKTIONEN DES APICOMPLEXA PHYLLUMS
COMPOSITION COMPRISING EXOPOLYSACCHARIDES EXTRACTED FROM PORPHYRIDIUM SP FOR THE TREATMENT AND/OR PREVENTION OF INFECTIONS BY THE PHYLUM APICOMPLEXA PARASITES

(30) Priorité: 21.12.2021 FR 2114179
(43) Date de publication de la demande: 28.06.2023
(73) Titulaire: Algosource, 44602 Saint-Nazaire Cedex (FR)
(72) Inventeur: Lépine, Olivier, 44600 Saint-Nazaire (FR)
(74) Mandataire: Vidon Brevets & Stratégie

(56) Documents cités:
- EP-B1- 3 206 477
- ORTEGA-BARRIA EDUARDO ET AL: "A Toxoplasma Lectin-like Activity Specific for Sulfated Polysaccharides Is Involved in Host Cell Infection", JOURNAL OF BIOLOGICAL CHEMISTRY , vol. 274, no. 3 1 janvier 1999 (1999-01-01), pages 1267-1276, XP055938622, US ISSN: 0021-9258, DOI: 10.1074/jbc.274.3.1267 Extrait de l'Internet: URL:https://www.sciencedirect.com/science/ article/pii/S0021925819882327/pdfft?md5=fb 2e0124685d19840fb3c90bcd20bd8a&pid=1-s2.0- S0021925819882327-main.pdf
- ADAMS YVONNE ET AL: "Inhibition of Plasmodium falciparum Growth In Vitro and Adhesion to Chondroitin-4-Sulfate by the Heparan Sulfate Mimetic PI-88 and Other Sulfated Oligosaccharides", ANTIMICROBIAL AGENTS AND CHEMOTHERAPY , vol. 50, no. 8 1 août 2006 (2006-08-01), pages 2850-2852, XP055938627, US ISSN: 0066-4804, DOI: 10.1128/AAC.00313-06 Extrait de l'Internet: URL:https://journals.asm.org/doi/pdf/10.11 28/AAC.00313-06
- Arlov Øystein ET AL: "ETH Library Engineered Sulfated Polysaccharides for Biomedical Applications", , 10 mai 2021 (2021-05-10), page FP, 1-104, XP055938643, Extrait de l'Internet: URL:https://www.research-collection.ethz.c h/bitstream/handle/20.500.11850/484261/3/A rlov_sulfatedpsreview_final_resubmit_AFM.p df [extrait le 2022-07-05]
- SOANEN NASTASIA ET AL: "Improvement of exopolysaccharide production by Porphyridium marinum", BIORESOURCE TECHNOLOGY , vol. 213 1 août 2016 (2016-08-01), pages 231-238, XP055938651, AMSTERDAM, NL ISSN: 0960-8524, DOI: 10.1016/j.biortech.2016.02.075 Extrait de l'Internet: URL:https://www.sciencedirect.com/science/ article/pii/S0960852416302127/pdfft?md5=06 d1fcf9b5f6fb9854148aed314bb3ba&pid=1-s2.0- S0960852416302127-main.pdf
- GARGOUCH NESRINE ET AL: "Potential of Exopolysaccharide from Porphyridium marinum to Contend with Bacterial Proliferation, Biofilm Formation, and Breast Cancer", MARINE DRUGS , vol. 19, no. 2 27 janvier 2021 (2021-01-27), page 66, XP055938679, DOI: 10.3390/md19020066 Extrait de l'Internet: URL:https://www.ncbi.nlm.nih.gov/pmc/artic les/PMC7911520/pdf/marinedrugs-19-00066.pd f
- MCFADDEN GEOFFREY IAN ET AL: "The apicoplast: now you see it, now you don't", INTERNATIONAL JOURNAL OF PARASITOLOGY, PERGAMON PRESS, GB, vol. 47, no. 2, 20 octobre 2016 (2016-10-20), pages 137-144, XP029900861, ISSN: 0020-7519, DOI: 10.1016/J.IJPARA.2016.08.005
- VENKATESAN MANIGANDAN ET AL: "Antioxidant, anticoagulant and mosquitocidal properties of water soluble polysaccharides (WSPs) from Indian seaweeds", PROCESS BIOCHEMISTRY, ELSEVIER LTD, GB, vol. 84, 29 mai 2019 (2019-05-29), pages 196-204, XP085745211, ISSN: 1359-5113, DOI: 10.1016/J.PROCBIO.2019.05.029 [extrait le 2019-05-29]

## Description

### 1. Domaine de l'invention

Le domaine de l'invention est celui du traitement et/ou de la prévention d'infections parasitaires, notamment d'infections par les parasites du phylum (ou embranchement) *Apicomplexa.*

Plus précisément, l'invention concerne l'utilisation d'une composition comprenant des exopolysaccharides obtenus à partir d'extraits de *Porphyridium sp* pour le traitement et/ou la prévention d'infections par les parasites du phylum *Apicomplexa.*

### 2. Art antérieur

Les parasites sont des organismes vivants pathogènes pour l'Homme qui ont, au cours de leur cycle de développement, le besoin de parasiter (ou infecter) un autre organisme, lui induisant des dégradations de son état physiologique, contrairement à une symbiose qui est profitable pour les deux organismes vivants.

Parmi les parasites d'animaux, dont l'Homme, on retrouve les parasites de l'embranchement des Apicomplexes (ou *Apicomplexa*), nommés également sporozoaires.

On compte dans cet embranchement différentes espèces connues telles que *Plasmodium falciparum* (parasite responsable du paludisme, ou malaria), *Toxoplasma gondii* (parasite responsable de la toxoplasmose), *Eimeria sp.* (parasite responsable de la coccidiose), etc. Ces parasites possèdent tous de fortes similarités structurelles et cycliques, suggérant ainsi l'utilisation d'une stratégie thérapeutique commune à ce phylum.

*Plasmodium falciparum* est le parasite le mieux connu du phylum *Apicomplexa.* Ce parasite est responsable de 220 millions de cas de paludisme dans le monde en 2010. Les manifestations sont particulièrement sévères chez les enfants qui représentent plus de % des 650 000 décès recensés lors de cette même année. Ce parasite contamine l'Homme via une piqûre de moustique du genre Anophèle, qui le transporte à l'intérieur de ses glandes salivaires. Le parasite induit une fièvre paludique caractérisée par la dégradation des globules rouges.

À l'heure actuelle, les techniques de prévention de la maladie sont : l'utilisation d'antipaludéens lors d'un déplacement dans des zones à risques, la limitation des risques de piqûres de moustiques *via* une sensibilisation des populations, ainsi que l'emploi d'insecticides. Les traitements de référence antipaludéens sont efficaces et peu coûteux. La bithérapie à base de substances d'origine naturelle, telle que l'Artémisinine et la 4-amino quinoléine, guérit 98% des cas. Toutefois, l'apparition de résistance pousse à continuer à rechercher de nouvelles molécules pour le traitement et/ou la prévention de l'infection par ce parasite. L'obtention de nouvelles molécules d'origine naturelle s'est avérée pour le moment décevante, notamment pour des raisons de pharmacodynamie qui ne permet pas de reproduire *in vivo* un effet significatif chez l'Homme (« Natural products as starting points for future anti-malaria thérapies : going bock to our roots ? », Wells et al., Malaria Journal, 2011).

Chez l'animal, *Eimeria sp.*, est responsable de la coccidiose du poulet, dont les conséquences sanitaires et financières sont importantes. La lutte anticoccidienne du poulet repose sur une combinaison comprenant une rotation des antibiotiques, des mesures d'hygiène et des vaccins (« Control of avion coccidiosis : future and présent natural alternatives », Ouiroz-Castañeda et al., BioMed Research International, 2015). L'efficacité des traitements médicamenteux, notamment des antibiotiques utilisés est à pondérer avec l'émergence de résistances et la forte pression des consommateurs pour des méthodes alternatives plus naturelles. L'aromathérapie, l'homéopathie, la phytothérapie, l'usage de pré- ou probiotiques ont ainsi été proposés. Ces nouvelles méthodes proposent de nouveaux modes d'action complémentaires de renfort de la barrière ou de l'immunité intestinale (« Coccidiose in poultry : anticoccidial products, vaccines and other prévention strategies », Peek et al., Vetenary Quarterly, 2011). En revanche, la prévention de la transmission *d'Eimeria sp.* ne fait pas encore partie de l'arsenal thérapeutique.

*Toxoplasma gondii* est le parasite responsable de la toxoplasmose. Le cycle de vie de *Toxoplasma gondii* comprend une forme classique impliquant un hôte définitif (comme par exemple les félidés domestiques ou sauvages) et un hôte intermédiaire. Les sporozoïtes du parasite, développés au sein des oocystes de matières fécales félines, sont dispersés dans l'environnement, puis ingérés par l'hôte intermédiaire. Au sein du tube intestinal, la forme sporozoïte se modifie en forme d'invasion dite tachyzoïte. Cette phase est rapidement limitée par le système immunitaire, qui contient le parasite sous forme de bradyzoïte (« Lytic Cycle of Toxoplasma gondii : 15 Years Later », Blader et al., Animal Review of Microbiology, 2015). Ainsi, cette infection est en général pauci-symptomatique pour l'immunocompétent. Elle est pourtant la principale infection parasitaire d'origine alimentaire responsable d'hospitalisations (« Foodborne Infections in France », Vaillant et al. Foodborne Pathogens and Disease, 2005). Les manifestations les plus sévères surviennent chez les sujets les plus fragiles, en raison de la réactivation de la forme parasitaire quiescente ou du passage des barrières biologiques de la forme invasive du parasite. Elle est notamment responsable chez l'immunodéprimé infecté par le virus du VIH (Virus de l'immunodéficience Humaine) de la toxoplasmose cérébrale et chez le foetus de complications oculaires et cérébrales majeures (« Toxoplasmosis Snapshots : Global Status of Toxoplasma Gondii Seroprevalence and Implications for Pregnancy and Congénital Toxoplasmosis », Pappas et al., International Journal of Parasitology, 2009). La transmission materno-foetale (ou TMF) survient lors de primo-infection maternelle *per partum* et peut même précéder la grossesse de deux mois.

Le traitement de la toxoplasmose repose sur une association de pyriméthamine et de sulfadiazine, dont la tolérance est souvent mauvaise. D'autres molécules sont disponibles comme par exemple le dapsone, la lindosamie, la clindamycine, l'atovaquone... Elles présentent également un risque d'effets secondaires indésirables et ne sont pas spécifiques de la toxoplasmose, ni du cycle parasitaire (« Human Toxoplasmosis-searching for novel chemotherapeutics », Antezazk et al., Biomedicine & Pharmacotherapy, 2016). Les traitements prophylactiques durant la grossesse sont initiés en cas de séroconversion. Ils comprennent la spiramycine avant 18 semaines d'aménorrhée, ou une combinaison de médicaments au-delà. Malgré tout, le taux de transmission verticale, c'est-à-dire de la mère à l'enfant, reste à 10% (« Systematic review and meta-analysis of the efficiency of anti-Toxoplasma gondii medicines in humons », Wei et al., PLoS ONE, 2015).

À l'heure actuelle, les seules mesures préventives résident dans des précautions alimentaires et une limitation de contact avec les animaux hôtes permanents du parasite. Pourtant, la piste préventive semble une voie complémentaire à proposer devant les complications liées au retard de diagnostic. Or, elle est pour le moment peu exploitée, la recherche se focalisant sur de nouvelles molécules utiles à visée thérapeutique. Les vaccins sont en cours d'essai chez l'animal, visant notamment l'expression protéique de molécules d'adhésion (« Immune response and protective effect against chronic Toxoplasma gondii infection induced by vaccination with DNA vaccine encoding Profilin », Gao et al, BMC Infectious Diseases, 2018).

Les nouvelles pistes pharmacologiques antitoxoplasmiques sont le plus souvent des molécules de synthèse visant la machinerie intracellulaire (par exemple le réticulum endoplasmique, les mitochondries, l'apicoplaste), la membrane cellulaire, le micronème ou l'extrémité apicale. Pour le seul *Toxoplasma gondii,* près de 40 sites d'action différents sont testés (« A systematic review of in vitro and in vivo activities of anti-toxoplasma drugs and compounds », Montazeri et al., Frontiers in Microbiology, 2017).

La phase d'invasion de *Toxoplasma gondii* est un phénomène relativement conservé au sein du phylum *Apicomplexa.* En effet, ces protozoaires intracellulaires obligatoires présentent un mode de locomotion unique qui leur permet de franchir des barrières biologiques non permissives et de pénétrer activement dans leurs cellules hôtes. Cette motilité conditionne leur survie et est assurée par un complexe apical sécrétant des molécules reliées à un système d'actine et myosine qui vont reconnaitre des molécules exprimées par la cellule hôte telles que les glycosaminoglycanes sulfatés (GAGs). Les glycosaminoglycanes (GAG) sont des polysaccharides linéaires complexes exprimés dans les compartiments intracellulaires, à la surface des cellules et dans l'environnement extracellulaire où elles interagissent avec diverses molécules pour réguler de nombreux processus cellulaires impliqués dans la santé et la maladie. Les parasites utilisent les GAG à pratiquement tous les principaux points d'entrée pour favoriser leur attachement et leur invasion des cellules hôtes, leur déplacement d'une cellule à l'autre et pour se protéger des attaques immunitaires.

En d'autres termes, le phénomène de glissement permet au parasite de traverser les barrières biologiques et d'atteindre les tissus cibles. Il est suivi d'une phase d'attachement passant également par la reconnaissance de GAGs (« Toxoplasma gondii uses sulfated proteoglycans for substrate and host cell attachment », Carruthers et al. Infection and Immunity, 2000). Les protéines du parasite impliquées dans les interactions avec les GAGs des cellules hôtes sont respectivement les micronèmes (par exemple MIC1 et 2) (« Toxoplasma gondii : microneme protein MIC 2 », Brossier et al., The International Journal of Biochemistry & Cell Biology, 2005), sécrétés lors de la phase de glissement au niveau de la partie apicale du parasite, et les antigènes de surface présents sur l'ensemble de la surface du parasite (par exemple SAG1, « *Surface Antigen 1* ») pour l'étape d'attachement. Ces familles de molécules d'adhésion sont différentes selon le parasite, mais s'avèrent des cibles thérapeutiques stratégiques préventives dans la lutte antiparasitaire du phylum *Apicomplexa* (« Toxoplasma gondii uses sulfated proteoglycans for substrate and host cell attachment », Carruthers et al. Infection and Immunity, 2000). En effet, les mécanismes impliqués dans la motilité des Apicomplexes, leur attachement et leur invasion de la cellule hôte sont en grande partie conservés à travers les membres du phylum.

Øystein et al., Engineered Sulfated Polysaccharides for Biomedical Applications, ETH Library Zurich, 2021, pages 1-14, retrouvé sur https://www.research-collection.ethz.ch/bitstream/ handle/20.500.11850/484261/3/Arlov_sulfatedpsreview_final_resubmit_AFM.pdf, démontré que les sulfates de dextran réduisent l'infectivité de Toxoplasma gondii, vraisemblablement en antagonisant les lectines qui se lient normalement aux polysaccharides sulfatés dans la matrice extracellulaire.

Il existe donc un besoin d'une nouvelle solution thérapeutique pour empêcher, ou pour le moins réduire, une infection induite par des parasites appartenant à l'embranchement des Apicomplexes. Notamment, il existe un besoin d'une nouvelle solution thérapeutique dont les effets secondaires indésirables sont réduits par rapport au traitements actuels.

### 3. Objectifs de l'invention

L'invention a notamment pour objectif de pallier aux inconvénients de l'art antérieur et de proposer une alternative thérapeutique à l'utilisation des composés médicamenteux actuels dans le traitement d'une infection par les parasites du phylum *Apicomplexa.*

Plus précisément, l'invention a pour objectif, dans au moins un mode de réalisation, de fournir une composition permettant de prévenir et/ou d'éliminer, ou à tout le moins réduire davantage, les infections par les parasites du phylum *Apicomplexa,* et notamment les infections par *Toxoplasma gondii.*

Un autre objectif de l'invention, dans au moins un mode de réalisation, est de fournir une composition permettant de prévenir et/ou d'éliminer, ou à tout le moins de réduire, les infections par *Toxoplasma gondii,* tout en améliorant la tolérance au traitement, c'est-à-dire en limitant les effets secondaire indésirables liés à la prise d'un traitement anti-infectieux.

### 4. Exposé de l'invention

Ces objectifs, ainsi que d'autres qui apparaîtront par la suite, sont atteints à l'aide d'une composition pour utilisation dans le traitement et/ou la prévention d'une infection parasitaire causée par un parasite appartenant au phylum *Apicomplexa.* Cette composition comprend un extrait obtenu à partir d'une microalgue rouge appartenant à l'espèce *Porphyridium sp,* ledit extrait comprenant des exopolysaccharides obtenus à partir de cette microalgue rouge.

L'invention propose ainsi une approche tout-à-fait nouvelle et inventive pour traiter et/ou prévenir une infection parasitaire qui consiste à utiliser des molécules issues de la biodiversité marine pour mimer les GAGs des cellules hôtes reconnues par le parasite et ainsi empêcher ou pour le moins limiter leur infection par ce dernier.

Contrairement à l'art antérieur (« Dual and Antagonic therapeutic effects of sulfated glycans », Pomin et al. Bioorganic & Medicinal Chemistry, 2016), qui ne considère que des molécules, parfois impures, d'origine animale, comme source de molécules mimant les GAGs (comme par exemple le kératane sulfate, l'héparane sulfate ou le chondroïtine sulfate), l'invention propose d'exploiter les ressources marines pour renforcer l'arsenal thérapeutique antiparasitaire, et en particulier les microalgues rouges qui sont intéressantes à plusieurs points de vue.

En effet, les microalgues rouges présentent un double avantage permettant d'envisager leur exploitation pharmaceutique à destination de sujet fragiles : une structure moléculaire plus régulière que les macroalgues, assurant ainsi un produit de composition stable (« Marine medicinal glycomics », Pomin et al., Frontiers in Cellular and Infection Microbiology, 2014) ; et une culture en photobioréacteur stérile. Il convient de noter que les différentes espèces de microalgues produisent des types différents de polysaccharides. En particulier, les microalgues rouges ne produisent pas de polysaccharides fibrillaires, mais des exopolysaccharides qui forment un mucilage de protection et encapsulent les cellules. Ce mucilage comprend une fraction d'exopolysaccharides (EPS) soluble dans le milieu, alors que 50 à 70% du polymère produit par la microalgue reste autour de la cellule pour former un mucilage qui encapsule cette dernière. Ces exopolysaccharides (EPS) solubles correspondent à la partie extraite et purifiée après mise en culture des microalgues (« Séparation and fractionation of exopolysaccharides from Porphyridium cruentum », Patel et al., Bioresource Technology, 2013).

En particulier, l'invention propose d'utiliser, comme souche productrice d'exopolysaccharides sulfatés, une microalgue rouge particulière, *Porphyridium sp,* dont le potentiel d'activité « GAG mimétique » n'a pas encore été exploré. Un avantage de cette algue rouge et, en particulier, de la souche *Porphyridium cruentum* est qu'elle est considérée comme conforme aux normes de sécurité définies par les autorités européennes. Ses EPS, composés majoritairement de xylose, galactose, glucose, acide glucuronique et rhamnose, ne sont aujourd'hui exploités que par l'industrie cosmétique pour leurs propriétés rhéologiques (« Red microalgal cell-wall polysaccharides : biotechnological aspects », Arad et al., Current Opinion in Biotechnology, 2010 ; « Superior biolubricant from a species of red microalgal », Arad et al, Langmuir, 2006 ; « Bioactivity and Applications of sulphated polysaccharides from marine microalgae », Jesus de Raposo et al., Marine Drugs, 2013).

Avantageusement, les inventeurs ont montré que les exopolysaccharides (ou EPS) obtenus à partir de microalgues rouges telles que *Porphyridium sp,* permettent une diminution de la croissance parasitaire. En particulier, les parasites du phylum *Apicomplexa* se sont avérés particulièrement sensibles à ce type de molécules.

Ainsi, l'utilisation de composés biomarins comme les EPS de *Porphyridium sp,* telle que proposée par l'invention, ouvre la voie à de nouvelles méthodes thérapeutiques dans le cadre du traitement d'infections parasitaires et notamment le traitement et/ou la prévention d'infections causées par les parasites issus du phylum *Apicomplexa.*

En outre, les inventeurs ont pu établir que l'utilisation des EPS n'induit pas de cytotoxicité pour les cellules hôtes. Ainsi, un traitement antiparasitaire à base d'EPS offre une alternative aux traitements actuels qui induisent parfois des effets secondaires indésirables, notamment dans le cas des traitements médicamenteux anti-toxoplasmiques.

Selon une autre caractéristique, la microalgue de l'espèce *Porphyridium sp* est choisie parmi : *Porphyridium cruentum* et *Porphyridium marinum.*

De manière avantageuse, l'utilisation de l'une ou l'autre de ces deux souches de *Porphyridium sp.* (*Porphyridium cruentum* et *Porphyridium marinum),* permet d'obtenir des exopolysaccharides pouvant être utilisés dans le traitement et/ou la prévention d'infections parasitaires. En effet, ces souches sont de bonnes sources d'approvisionnement en EPS.

Selon une autre caractéristique, l'extrait comprend entre 50 et 200 µg/mL d'exopolysaccharides extrait de *Porphyridium cruentum* ou *Porphyridium marinum.*

Selon une autre caractéristique, le parasite appartient au phylum *Apicomplexa* est *Toxoplasma gondii.*

Selon une caractéristique de l'invention, la microalgue de l'espèce *Porphyridium sp* est choisie parmi : *Porphyridium cruentum* et *Porphyridium marinum.*

Selon une autre caractéristique, l'infection parasitaire est causée par un parasite appartenant au phylum *Apicomplexa,* ledit parasite étant *Toxoplasma gondii.*

Selon une autre caractéristique de l'invention, l'extrait comprend entre 50 et 200 µg/mL d'exopolysaccharides extrait de *Porphyridium cruentum* ou *Porphyridium marinum.*

### 5. Brève description des figures

D'autres buts, caractéristiques et avantages de l'invention apparaîtront plus clairement à la lecture de la description suivante, donnée à titre de simple exemple illustratif, et non limitatif, en relation avec les figures, parmi lesquelles :
[Fig. 1] : la figure 1 illustre un schéma d'une structure cellulaire d'un sporozoïte ou mérozoïte des parasites provenant de l'embranchement des Apicomplexes ;
[Fig. 2] : la figure 2 présente sous forme de diagramme un exemple de procédé de culture de microalgue et de production et récupération d'ExoPolySaccharides de la microalgue *Porphyridium cruentum ;*
[Fig. 3] : la figure 3 représente un histogramme illustrant la viabilité de cellules HFF après traitement par une composition comprenant des exopolysaccharides extrait de *Porphyridium cruentum* ou *Porphyridium marinum ;*
[Fig.4] : la figure 4 représente un histogramme illustrant le développement parasitaire après infection par *Toxoplasma gondii* de cellules HFF et traitement par une composition comprenant des exopolysaccharides extrait de *Porphyridium cruentum* ou *Porphyridium marinum.*

### 6. Description des modes de réalisation de l'invention

Les parasites ayant montré des sensibilités envers des molécules de *Porphyridium sp,* sont en particulier ceux de l'embranchement des Apicomplexes.

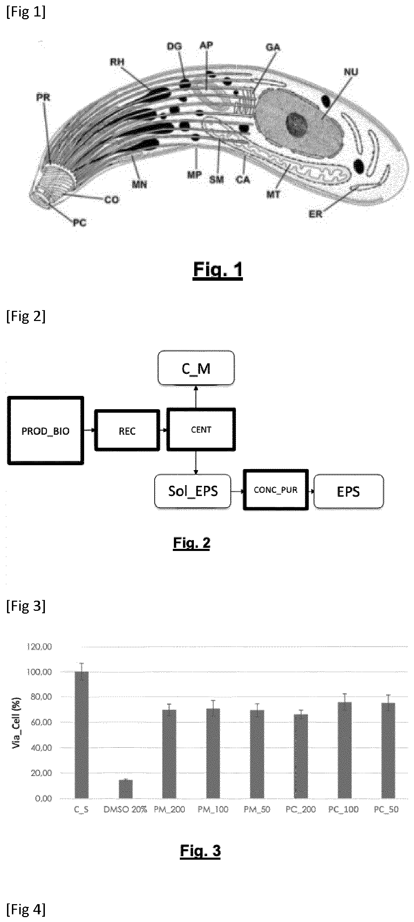

La figure 1 représente un schéma d'une structure cellulaire des parasites du phylum *Apicomplexa.* En particulier, la forme sporozoïte et la forme mérozoïte sont retrouvées chez l'Homme. Les oocystes caractérisent la forme contaminante des parasites trouvés dans l'environnement. Les oocystes sont des « oeufs » qui sont la forme de propagation des parasites du phylum Apicomplexa dans l'environnement extérieur et donc de l'infection parasitaire. La forme sporozoïte et la forme merozoïte comprennent un apicoplaste AP, des alvéoles corticales CA, une zone comoïde CO, des denses granules DG, un réticulum endoplasmique ER, un appareil de golgi GA, des micronèmes MN, un micropore MP, des mitochondries MT, un noyau NU, des anneaux pré-conoïdales PC, un anneau polaire PR, des rhoptries RH et des microtubules subpelliculaires SM.

Afin de démontrer l'effet antiparasitaire des EPS de *Porphyridium sp.,* des tests *in vitro* d'activités antitoxoplasmiques ont été réalisés sur des fibroblastes de prépuce humain HFF (« *Human Foreskin Fibroblasts* ») infestés par une souche de *Toxoplasma gondii* génétiquement modifiée pour exprimer une β-D-galactosidase. L'expression de cette enzyme permet de quantifier la croissance parasitaire en présence d'un substrat (par exemple du chlorophénole rouge-β-D-galactosidase ou CPRG) (« Use of Toxoplasma gondii expressing β-galactosidase for colorimétrie assessments of drug activity in vitro », Mc Fadden et al, Antimicrobial and Chemotherapy, 1997).

Les exopolysaccharides (EPS) de *Porphyridium marinum* (PM) et *Porphyridium cruentum* (PC) ont été testés après 1h de contact avec le parasite.

### 6.1. Cultures des microalgues rouges

Les souches microalgales utilisées sont les suivantes :
- *Porphyridium cruentum* (PC) : UTEX 161 ;
- *Porphyridium marinum* (PM) : Kylin (1937) CCAP 1380/10.

Toutes les souches de *Porphyridium sp* produisent des polysaccharides identiques, et en particulier des EPS, et l'activité démontrés n'est pas spécifique à une espèce.

La mise en culture des microalgues est effectuée dans un milieu adapté : typiquement un milieu d'Hemerick modifié.

Le tableau 1 ci-dessous présente la composition du milieu d'Hemerick modifié (MHM5) utilisé pour la culture des microalgues.

**[Table 1]**

| Macroéléments | Concentration (g/L) | Microéléments | Concentration (mg/L) |
|---|---|---|---|
| NaCl | 29 | H3BO3 | 2 |
| MgSO4, 7H2O | 6 | MnCL2, 4H2O | 1,8 |
| NaNO3 | 1,5 | (NH4)6Mo7O24, 4H2O | 0,02 |
| CaCL2, 2H2O | 0,294 | ZnSO4, 7H2O | 0,213 |
| KCL | 0,75 | CuSO4, 5H2O | 0,08 |
| NaHCO3 | 1,7 | CoSO4, 7H2O | 0,91 |
| KH2PO4 | 0,8 | | |
| FeEDTA | 0,05 | | |

On présente désormais en lien avec la figure 2 un exemple de procédé de culture d'une microalgue *Porphyridium cruentum* et d'extraction des EPS produits par celle-ci.

Il est à noter que la même procédure peut être mise en oeuvre avec la microalgue *Prophyridium marinum.*

Tous les systèmes de culture de microalgues permettant de cultiver *Porphyridium sp* sont adaptés : bassin de type Raceway, photo-bioréacteur tubulaire, plan, hélicoïdale...

Dans l'exemple en lien avec la figure 2, la mise en culture, c'est-à-dire la production PROD_BIO de biomasse et donc des EPS, est réalisée en photobioréacteur (PBR) de type Air-Lift tubulaire de 10L à 25°C, inoculé par une culture antérieure en PBR Air-Lift plan d'un litre associé à un milieu d'Hemerick modifié.

La production d'EPS débute à la fin de la phase exponentielle de développement des microalgues et se majore à la phase stationnaire de développement des microalgues.

Les EPS sont excrétées dans le milieu de culture après une phase de maturation des microalgues, le surnageant de culture est alors recueilli par centrifugation CENT suite à l'étape de récolte REC de la biomasse. Les exopolysaccharides en solution Sol_EPS sont séparés des cellules microalgales C_M et extraits.

Plusieurs technologies peuvent être utilisées pour la concentration et la purification CONC_PUR de la solution d'EPS Sol_EPS obtenue après centrifugation, pour obtenir des EPS purifiés, comme la filtration membranaire par diafiltration / ultrafiltration et la précipitation à l'éthanol.

Dans une variante, les polysaccharides, et en particulier les EPS, peuvent aussi être extraits à partir de la biomasse qui en contient encore (jusqu'à 20%). Plusieurs technologies sont possibles : par broyage à bille selon une technique décrite dans le brevet EP3206477B1, ou cavitation ou encore extraction à l'eau chaude... Quelle que soit la technologie d'extraction, les polysaccharides, comme les EPS, non hydrolysés, peuvent rentrer dans la composition objet de la présente invention.

### 6.2. Mesure de l'activité anti-infectieuse de Toxoplasma gondii

La mesure de l'activité anti-infectieuse est basée sur une méthode mise au point au laboratoire LMGE (« Laboratoires Microorganismes, Génomes et Environnement »). La méthode est décrite ci-dessous.

### 6.2.1 La souche parasitaire

La souche de *Toxoplasma gondii* cultivée au sein du laboratoire est une souche de parasite génétiquement modifiée bien connue : le gène d'expression de l'enzyme de β-D-galactosidase a été inséré dans le génome du parasite. Ainsi, il est possible par le dosage de l'activité de cette enzyme de connaître la viabilité et la croissance des parasites. En présence du substrat de l'enzyme, le CPRG, il y a formation d'un complexe coloré qui est quantifié par spectroscopie à 550 nm.

### 6.2.2 Analyse de la viabilité cellulaire

L'effet des EPS est évalué, dans un premier temps, sur des cellules HFF qui correspondent aux cellules-hôtes de *Toxoplasma gondii.* La méthode MTT (« Towards an optimized MTT assay », Niks et al, Journal of Immunological Methods, 1990) est utilisée pour évaluer l'impact des EPS produit par les souches de microalgues PM et PC sur la viabilité des cellules HFF.

Le sel de térazolium MTT (bromure de 3-(4,5-dimethylthiazol-2-yl)-2,5-diphenyl tetrazolium) est un composé soluble dans l'eau, donnant une solution jaune. Il pénètre les cellules *a priori* par endocytose. L'anneau de tétrazolium contenu dans le MTT est réduit en formazan par le succinate déshydrogénase mitochondriale des cellules vivantes actives, formant un précipité insoluble dans la mitochondrie de couleur violette, dit aussi « cristaux de formazan ». Aussi, les cellules mortes ne peuvent pas former de cristaux. La quantité de précipité formée est donc proportionnelle à la quantité de cellules vivantes, mais surtout à l'activité métabolique de chaque cellule. Ainsi, après l'incubation des cellules en présence de MTT pendant environ 2 à 3H à 37°C, les cellules et les cristaux sont dissous à l'aide d'un solvant (par exemple DMSO pour diméthylsulfoxyde, ou isopropanol). La coloration est mesurée par spectroscopie pour connaître la quantité relative de cellules vivantes et surtout, actives métaboliquement.

Les conditions sont les suivantes :
- Contrôle négatif : cellules saines non traitées (C_S) ;
- Contrôle positif : cellules traitées au DMSO 20% (DMSO 20%) ;
- Cellules HFF traitées 4 jours en présence d'extraits issus des microalgues PM et PC avec des concentrations en EPS de 50µg/mL, 100µg/mL et 200µg/mL (PM_200 ; PM_100; PM_50 ; PC_200 ; PC_100 ; PC_50).

Les résultats sont présentés en lien avec la figure 3 décrite ci-dessous en section 6.3.1.

### 6.2.3 Analyse de la croissance de Toxoplasma gondii : tests anticoccidiens

Les tachyzoïtes (formes infectieuses de *Toxoplasma gondii*) sont pré-traités avec des extraits comprenant des exopolysaccharides(EPS) obtenus selon le procédé décrit en lien avec la figure 2, à partir des souches de microalgues PM et PC, ainsi que les cellules-hôtes HFF non infectées par *Toxoplasma gondii* pendant 1h.

Un fois arrivées à confluence dans des plaques de culture 96 puits, les cellules-hôtes HFF sont ensuite infectées par 2.10⁴ tachyzoïtes pré-traités (soit 200µL) pendant 1 heure. Celles-ci sont ensuite lavées une fois avec du milieu MEM (« *Minimum Essential Medium* », ou milieu de culture minimum essentiel en français) supplémenté.

Les extraits comprenant des EPS obtenus à partir des souches de microalgues PM et PC à tester sont ensuite ajoutés à la bonne concentration d'EPS dans un volume de 200µL.

Trois témoins sont réalisés : cellules HFF saines (non représentées), cellules HFF infectées non traitées (C_IN) et cellules HFF infectées et traitées à la pyriméthamine (molécule anticoccidienne de référence)(Pyr). Au bout de 72 heures, la croissance du parasite *T.gondii* est évaluée en dosant l'activité β-D-galactosidase des parasites, le dosage étant proportionnel au nombre de parasites. Les plaques 96 puits sont ainsi centrifugées à 265 x g pendant 5 min. Les cellules sont lysées avec 50µL de tampon de lyse pendant 1h à 50°C, après avoir ôté le milieu du culture. Le tampon de réaction (160µL) et ensuite ajouté et les plaques sont incubées 5 min à 37°C afin d'optimiser l'activité de l'enzyme β-D-galactosidase. Ensuite, le substrat de l'enzyme : rouge de chlorophénol β-D galactosidase (mis préalablement 1h à 50°C) à une concentration de 6,25mM en solution dans du tampon phosphate pH=7,3, est ajouté (40µL). Les plaques de culture sont incubées 30 min jusqu'à obtention d'une coloration rouge. La DO (Densité Optique) ou absorbance, à 550nm est mesurée au lecteur de micro-plaques pour quantifier la croissance parasitaire.

Des tests de comparaison de moyennes sont effectués et en fonction de la normalité des données de tests paramétriques (par exemple de type Anova) ou non paramétriques (par exemple Kruskall et Wallis) sont effectués pour vérifier la significativité des résultats.

Les conditions expérimentales sont les suivantes :
- Cellules saines (non représentées)
- Cellules infectées non traitées (C_IN)
- Cellules infectées + pyriméthamine 0,1µg/mL (molécule anticoccidienne de référence)(Pyr)
- Cellules infectées + extrait de PM 200µg/mL (PM_200)
- Cellules infectées + extrait de PM 100µg/mL (PM_100)
- Cellules infectées + extrait de PM 50µg/mL (PM_50)
- Cellules infectées + extrait de PC 200µg/mL (PC_200)
- Cellules infectées + extrait de PC 100µg/mL (PC_100)
- Cellules infectées + extrait de PC 50µg/mL (PC_50)

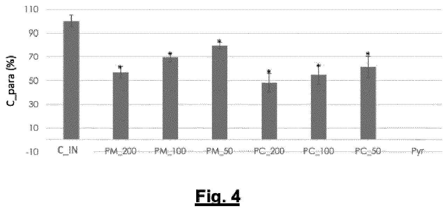

Les résultats sont présentés en lien avec la figure 4 décrite ci-dessous en section 6.3.2.

### 6.3. Résultats

Une réduction de la croissance parasitaire dose dépendante a été constatée pour les deux souches de *Porphyridium sp :* réduction de 43% pour *Porphyridium cruentum* (PC) et 52% pour *Porphyridium marinum* (PM) à 200µg/mL d'EPS, sans toxicité cellulaire.

### 6.3.1. Résultats sur l'analyse de la viabilité cellulaire

Comme décrit ci-dessus en section 6.2.2, préalablement aux essais sur parasite, un test MTT est réalisé pour savoir si les extraits de PC et PM sont toxiques pour les cellules-hôtes HFF permettant la croissance de *T.gondii.* Autrement dit, le test MTT vise à démontrer l'absence d'effet important des extraits de PM et PC sur la viabilité des cellules-hôtes HFF.

Comme illustré en figure 3, les extraits de PM et PC entraînent une légère baisse de la viabilité des cellules-hôtes, mais pas d'effet cytotoxique (observations effectuées au microscope).

L'histogramme de la figure 3 représente en ordonnée le pourcentage de viabilité cellulaire Via_Cel (%) et en abscisse les différentes conditions de traitements aux EPS comme présenté précédemment.

### 6.3.2. Résultats sur la croissance de T.gondii : activité anticoccidienne des exopolysaccharides

La figure 4 illustre l'effet antiparasitaire des EPS extraits des souches de microalgues PC ou PM à différentes concentrations. Il est d'abord constaté que la pyriméthamine (Pyr) a bien un effet antiparasitaire, puisque nous n'observons pas de croissance de *T.gondii.*

En ce qui concerne les extraits de PM et PC testés, les activités anticoccidiennes les plus importantes sont observées avec les concentrations les plus fortes en exopolysaccharides, avec des réductions significatives de la croissance parasitaire de 43% pour des extraits de PC à 200µg/mL d'EPS et 52% pour des extraits de PM à 200µg/mL d'EPS.

Un effet dose-réponse est à noter puisque l'activité des exopolysaccharides diminue quand leur concentration diminue. Toutefois, les prétraitements des parasites n'ont pas permis de bloquer totalement l'infection des cellules.

L'histogramme de la figure 4 représente en ordonnée le pourcentage de croissance parasitaire C_para (%) et en abscisse les différentes conditions de traitements aux EPS comme présenté précédemment.

### 6.4 Discussion

L'invention qui vient d'être présentée constitue la première description d'activité antiparasitaire contre des parasites du système digestif humain pour les EPS microalgaux. Les résultats obtenus ouvrent des perspectives nouvelles de chimio-prévention pouvant faire envisager une place pour l'industrie microalgale au sein d'une stratégie pharmaceutique innovante. On note qu'il pourrait en outre être particulièrement intéressant de confirmer la cible protéique et valider l'efficacité et la tolérance des EPS microalgaux *in vivo.*

Les résultats qui viennent d'être décrits permettent de mettre en évidence une activité antiparasitaire des EPS de *Porphyridium sp.* Les produits, ou extraits comprenant des EPS obtenus à partir de PM et PC, présentent une activité anticoccidienne de l'ordre de 50%, avec un prétraitement des parasites pendant une heure. Il serait dont intéressant de tester plusieurs temps de prétraitement des parasites (par exemple 30min, 1h, 2h). L'augmentation du temps de prétraitement devrait augmenter ainsi l'activité des produits obtenus à partir de PM et PC.

Il s'agit aussi de la première étude d'activité d'un composé d'origine marine visant *Toxoplasma gondii.* Ces résultats confirment l'intérêt potentiel des polysaccharides sulfatés microalgaux, notamment des exopolysaccharides, à visée anti-infectieuse (« Bioactivity and Applications of sulphated polysaccharides from marine microalgae », Jesus de Raposo et al., Marine Drugs, 2013,).

Afin de valider que le mécanisme d'inhibition de l'activité parasitaire est lié à une activité « GAG mimétique » des EPS de *Porphyridium cruentum* et *Porphyridium marinum,* il serait intéressant de confirmer l'interaction des EPS avec une ou des molécule(s) d'adhésion du pathogène et d'exclure des faux positifs associés au solvant de purification.

Ainsi, un avantage de choisir des microalgues rouges est qu'elles sont d'ores et déjà reconnues comme sûres pour l'hôte (« Microalgae-based product for the food and feed sector : an outlook for Europe », Enzig et al., 2016), ce qui permet d'envisager une potentielle bonne tolérance de l'hôte.

Les activités anti-infectieuses des polysaccharides microalgaux testées jusqu'à maintenant se sont portées préférentiellement vers les virus (« Bioactivity and Applications of sulphated polysaccharides from marine microalgae », Jesus de Raposo et al.). Elles tiennent compte d'interactions *in vitro* entre la molécule et le virus, mais excluent le phénotype de la cellule hôte et/ou de la barrière tissulaire à traverser. En général, ces virus visent des ligands spécifiques dont les GAGs (« Glycosaminoglycans and infection », Park et al., Frontiers in bioscience, 2016), dont l'expression varie selon le tissu et explique les modes de transmission virale différents. Au contraire avec l'invention il est possible de prendre en compte le mode d'invasion du pathogène de l'étage moléculaire à tissulaire, et la sécurité de l'hôte.

Au vu des résultats obtenus , et en particulier des données sur la tolérance qui ont été confirmées à l'échelle cellulaire dans cette étude, *Porphyridium cruentum* paraît le candidat idéal pour son application rapide. Son statut hybride anti-infectieux à visée préventive risque de faire basculer son indication règlementaire vers le champ médical.

Par conséquent, ces résultats donnent l'opportunité idéale d'adapter la méthode pharmacologique aux futurs nutricaments ou médicaments microalgaux. L'invention pourrait s'intégrer avantageusement dans une stratégie thérapeutique anti-toxoplamique définie, comme par exemple la chimio-prévention. Cette stratégie ne détermine pas encore vers quel hôte elle sera la plus active. Le choix de destination vers le chat modèle animal hôte définitif permettrait de limiter la transmission à l'Homme, mais n'éliminerait pas les principales voies de contamination qui passent par les animaux non domestiques. La formulation vers l'Homme devra résoudre des questions de tolérance en raison de la fragilité des sujets exposés. Enfin, il pourrait aussi être intéressant de surveiller l'apparition de résistances en vue de la mise en application de cette nouvelle stratégie de chimioprévention (« Multidrug résistance : an emerging crisis », Tanwar et al., Interdisciplinary Perspectives on Infectious Diseases, 2014).

L'invention qui vient d'être décrite procure de nombreux avantages. Elle présente des résultats prometteurs pour le traitement et/ou la prévention d'infections parasitaires, ce qui permet d'envisager l'entrée de nouveaux composés bioactifs issus des polysaccharides marins (comme les EPS) sur le marché pharmaceutique.

## Revendications

1. Composition pour utilisation dans le traitement et/ou la prévention d'une infection parasitaire causée par un parasite appartenant au phylum *Apicomplexa,* ladite composition comprenant un extrait obtenu à partir d'une microalgue rouge appartenant à l'espèce *Porphyridium sp,* ledit extrait comprenant des exopolysaccharides obtenus à partir de ladite microalgue rouge.

2. Composition pour utilisation selon la revendication 1, **caractérisé en ce que** ladite microalgue de l'espèce *Porphyridium sp* est choisie parmi : *Porphyridium cruentum* et *Porphyridium marinum.*

3. Composition pour utilisation selon la revendication 2, **caractérisé en ce que** ledit extrait comprend entre 50 et 200 µg/mL d'exopolysaccharides extrait de *Porphyridium cruentum* ou *Porphyridium marinum.*

4. Composition pour utilisation selon l'une quelconque des revendications 1 à 3, **caractérisé en ce que** ledit parasite appartenant au phylum *Apicomplexa* est *Toxoplasma gondii.*

## Patentansprüche

1. Zusammensetzung zur Verwendung bei der Behandlung und/oder Vorbeugung einer durch einen zum Stamm *Apicomplexa* gehörenden Parasiten verursachten Parasiteninfektion, wobei die Zusammensetzung einen Extrakt umfasst, der aus einer zur Spezies *Porphyridium sp*. gehörenden roten Mikroalge erhalten wird, wobei der Extrakt Exopolysaccharide umfasst, die aus der roten Mikroalge gewonnen werden.

2. Zusammensetzung zur Verwendung nach Anspruch 1, **dadurch gekennzeichnet, dass** die Mikroalge der Spezies *Porphyridium sp.* aus *Porphyridium cruentum* und *Porphyridium marinum* ausgewählt ist.

3. Zusammensetzung zur Verwendung nach Anspruch 2, **dadurch gekennzeichnet, dass** der Extrakt zwischen 50 und 200 µg/ml aus *Porphyridium cruentum* oder *Porphyridium marinum* extrahierte Exopolysaccharide enthält.

4. Zusammensetzung zur Verwendung nach einem der Ansprüche 1 bis 3, **dadurch gekennzeichnet, dass** der zum Stamm *Apicomplexa* gehörende Parasit *Toxoplasma gondii* ist.

## Claims

1. A composition for use in the treatment and/or prevention of a parasitic infection caused by a parasite belonging to the phylum Apicomplexa, said composition comprising an extract obtained from a red microalgae belonging to the species Porphyridium sp, said extract comprising exopolysaccharides obtained from said red microalgae.

2. The composition for use according to claim 1, **characterised in that** said microalgae of the species Porphyridium sp is selected from: Porphyridium cruentum and Porphyridium marinum.

3. The composition for use according to claim 2, **characterised in that** said extract comprises between 50 and 200 µg/mL of exopolysaccharides extracted from Porphyridium cruentum or Porphyridium marinum.

4. The composition for use according to any one of claims 1 to 3, **characterised in that** said parasite belonging to the phylum Apicomplexa is Toxoplasma gondii.
